# EUROPÄISCHE PATENTSCHRIFT

(11) **EP 2 825 108 B1**
(45) Veröffentlichungstag und Bekanntmachung des Hinweises auf die Patenterteilung: **06.11.2019**
(21) Anmeldenummer: 13828778.4
(22) Anmeldetag: 27.11.2013
(51) Int. Cl.: A61B 17/3205, A61B 17/3207, A61F 9/007

(54) **INSTRUMENT ZUR STIMULATION BZW. IRRITATION UND/ODER ZUR ABRASIVBEHANDLUNG UND/ODER ZUM POLIEREN EINER MEMBRAN ODER OBERFLÄCHE ODER INNENFLÄCHE IM MENSCHLICHEN ODER TIERISCHEN AUGE**
INSTRUMENT FOR STIMULATING OR IRRITATING AND/OR ABRASIVELY TREATING AND/OR POLISHING A MEMBRANE OR SURFACE OR INNER SURFACE IN THE HUMAN OR ANIMAL EYE
INSTRUMENT DE STIMULATION OU D'IRRITATION ET/OU DE TRAITEMENT PAR ABRASION ET/OU DE POLISSAGE D'UNE MEMBRANE OU D'UNE SURFACE EXTÉRIEURE OU D'UNE SURFACE INTÉRIEURE DE L'OEIL D'UN HOMME OU D'UN ANIMAL

(30) Priorität: 13.12.2012 DE 102012223076
(43) Veröffentlichungstag der Anmeldung: 21.01.2015
(73) Patentinhaber: Geuder AG, 69126 Heidelberg (DE); Rheinische Friedrich-Wilhelms-Universität Bonn, 53113 Bonn (DE)
(72) Erfinder: BRAUN, Norbert, 74909 Meckesheim (DE); STANZEL, Boris V., 53127 Bonn (DE); HOLZ, Frank G., 53127 Bonn (DE); BRINKEN, Ralf, 53119 Bonn (DE)
(74) Vertreter: Patent- und Rechtsanwälte Ullrich & Naumann
(86) Internationale Anmeldenummer: PCT/DE2013/200320
(87) Internationale Veröffentlichungsnummer: WO 2014/090244

(56) Entgegenhaltungen:
- WO-A1-2011/097578
- DE-A1-102011 100 371
- US-A- 5 057 114
- US-A1- 2005 267 490
- US-A1- 2008 183 199
- US-B1- 6 575 989

## Beschreibung

Die Erfindung betrifft ein Instrument zur Stimulation bzw. Irritation und/oder zur Abrasivbehandlung und/oder zum Polieren einer Membran oder Oberfläche oder Innenfläche im menschlichen oder tierischen Auge, mit einem ein Betätigungsorgan umfassenden Handgriff und einer sich an den Handgriff anschließenden, am freien, distalen Ende offenen Sonde zum Einführen in das Auge.

Bei dem hier in Rede stehenden Instrument handelt es sich um ein Instrument zur Anwendung in der ophthalmologischen Chirurgie, insbesondere zur Vorbereitung des subretinalen Einsetzes eines Implantats zur Behandlung einer altersabhängigen Makuladegeneration (AMD). Der Ersatz von funktionsbeeinträchtigten, submakulären RPE-Zellen (Retinales Pigment-Epithel) kann sinnvolles Therapieziel für fortgeschrittene Formen der AMD sein, um nämlich die Homeostase des RPE-Photorezeptor-Komplexes wiederherzustellen. Letztendlich geht es darum, den Photorezeptoren ein funktionstüchtiges RBE unterzulegen. Eine Übersicht hierüber findet sich in Binder S, Stanzel BV, Krebs I, Glittenberg C. Transplantation oft he RPE in AMD. Prog Retin Eye Res 2007;26:516-554.

Instrumente zum Einsetzen eines subretinalen Implantats sind beispielsweise aus DE 10 2011 100 371 A1, DE 298 19 018 U1, EP 0 967 919 B1 und WO 00/76403 A1 bekannt.

Der Erfindung liegt folgende Überlegung zugrunde:
Das Einpflanzen von RPE Einzelschicht Transplantaten auf eine intakte RPE-Schicht eines Empfängers kann aberrante oder unerwünschte Signalkaskaden erzeugen. Auch stellt ein intaktes RPE eine Stoffwechselbarriere für das Transplantat dar. AMD-Patienten mit trockener intermediärer oder geographischer Atrophie mit fovealer Aussparung als potentielle Kandidaten für die RPE-Entfernung verfügen demnach über intaktes RPE im anzustrebenden Implantationsareal.

Es liegt die Vermutung zugrunde, dass durch atraumatische Entfernung des Wirt-RPE der Stoffwechsel des RPE-Transplantats durch Annäherung an die Bruch'sche Membran/ den Choriocapillaris-Komplex verbesserbar ist. Außerdem lassen sich dadurch Grundlagen für Verankerungsstrategien des Zellträgermaterials in der Extrazellulärmatrix schaffen. Durch diesen Ansatz können veraltete und ggf. degenerierte Zellen des Patienten durch ein vermeintlich gesundes RPE-Transplantat ersetzt und positionsstabilisiert werden.

Aus der Praxis sind Diamantstaub-beschichtete Silikon-Spatel bekannt, die jedoch bei abrasiver Anwendung Schäden in der Netzhaut und BM hervorrufen.

Ein ebenfalls aus der Praxis bekanntes hydraulisches RPE-Debridement kann zu RPE-Dispersion im Glaskörperraum und damit zu proliferativer Vitreoretinopathie führen. Außerdem kann ein zu kräftiger Flüssigkeitsstrahl oder eine Manipulation im subretinalen Raum selbst erhebliche Photorezeptorschäden hervorrufen.

Eine abrasive RPE-Abtragung mit einer Silikon- oder Metallkanüle führt aufgrund der direkten Fortleitung der vektoriellen Kraftausübung durch die Hand des Chirurgen zu Rissen und Schäden in der BM und Choriocapillaris. Eine EDTAunterstützte Entfernung des RPE eröffnet die "tight junctions" des RPE, womit zwar die Zell-Zell Adhäsion gelockert wird, jedoch kaum oder keine Auswirkung auf die RPE-BM Adhäsion entsteht. EDTA hat zudem nachweislich eine Photorezeptortoxizität, weshalb der Einsatz am Patienten, nach derzeitigem Wissensstand, ausgeschlossen erscheint.

Die selektive RPE-Destruktion durch geeignete Laser führt zu irreversiblen thermalen Schäden in angrenzenden Bereichen und ist außerdem aufgrund des apparativen Aufwands kostenintensiv.

Aufgrund der aus der Praxis bekannten Methoden und der dazu verwendeten Instrumenten und unter Berücksichtigung der durch die Anwendung solcher Instrumente verbundenen Nachteile liegt der Erfindung die Aufgabe zugrunde, ein Instrument zur Stimulation bzw. Irritation und/oder zur Abrasivbehandlung und/oder zum Polieren einer Membran oder Oberfläche oder Innenfläche im menschlichen oder tierischen Auge anzugeben, wonach bei einfachster Konstruktion eine atraumatische Entfernung des RPE möglich ist. Außerdem soll dieses Instrument geeignet sein, jedwede Membran bzw. Oberflächen im Auge stimulieren bzw. irritieren, abrasiv behandeln oder polieren zu können, je nach Bedarf, und dies auf schonende Weise, insbesondere auch in Bezug auf angrenzende Bereiche.

US 2008/183199 A1 offenbart ein Instrument zur Stimulation bzw. Irritation und/oder zur Abrasivbehandlung und/oder zum Polieren einer Membran oder Oberfläche oder Innenfläche im menschlichen oder tierischen Auge.

Die voranstehende Aufgabe ist durch ein Instrument gemäß Patentanspruch 1 gelöst. Danach ist ein solches Instrument dadurch gekennzeichnet, dass die Sonde im Inneren eine Schlinge umfasst, die über das Betätigungsorgan zumindest geringfügig aus der Sonde heraus schiebbar und in die Sonde zurück schiebbar bzw. ziehbar ist.

Bei dem erfindungsgemäßen Instrument handelt es sich um ein rein mechanisches Instrument mit verblüffend einfacher Konstruktion. Dieses Instrument hat einen Handgriff mit Betätigungsorgan und eine sich an den Handgriff anschließende, am distalen Ende offene Sonde, die zum Einführen in das Auge dient, nämlich zum Einführen in den mechanisch zu behandelnden Bereich bzw. bis hin zu der mechanisch zu behandelnden Oberfläche/Membran. Die Sonde umfasst in ihrem Inneren, beispielsweise in einem innerhalb der Sonde durchgehenden Kanal, eine Schlinge, die über das Betätigungsorgan zumindest geringfügig aus der Sonde heraus schiebbar und in die Sonde zurück schiebbar bzw. ziehbar ist. Beim Einführen in das Auge befindet sich die Schlinge im Innern der Sonde. Vor Ort, d.h. im eingeführten Zustand im Auge, kann die Schlinge aus der Sonde herausgefahren werden, wodurch die Schlinge ihre zur Bearbeitung erforderliche Größe und Form entfaltet. Nach Beendigung des Eingriffs lässt sich die Schlinge mühelos wieder in die Sonde hineinschieben, so dass die Sonde gefahrlos aus dem Auge gezogen werden kann.

Der hier realisierte Mechanismus ermöglicht es beispielsweise, dass nur die Schlinge in den subretinalen Raum eingeführt werden muss. Dies reduziert operativen Aufwand.

Bei der Schlinge handelt es sich in vorteilhafter Weise um einen dünnen Metall- oder Kunststoffdraht. Ganz besonders eignet sich eine Schlinge aus einem kristallinen Stereoisomer von Polypropylen. Im Konkreten kann es sich dabei um ein handelsübliches Nahtmaterial handeln, welches unter der Marke "PROLENE" auf dem Markt ist.

Die Schlinge ist äußerst dünn ausgeführt und kann einen Materialdurchmesser von etwa 0,06 bis 0,07 mm haben. Im herausgeschobenen Zustand, vom freien Ende der Sonde aus gemessen, kann die Schlinge eine Länge von 2 bis 4 mm, vorzugsweise von 3 mm haben. Ein Innendurchmesser von 1 bis 2 mm, vorzugsweise 1,6 mm, hat sich im Einsatz bewährt.

Gemäß der Erfindung, umfasst die Sonde ein äußeres Führungsrohr und ein endseitig die Schlinge tragendes Innenrohr, wobei das Innenrohr durch Betätigung des Betätigungsorgans innerhalb des Führungsrohrs verschiebbar ist. Durch die Verschiebung des die Schlinge tragenden Innenrohrs lässt sich die Schlinge aus dem Führungsrohr heraus und in das Führungsrohr hinein verlagern, je nach Verschieberichtung. Mit anderen Worten ist hier ein einfacher Betätigungsmechanismus geschaffen, der die am Innenrohr befestigte Schlinge durch Verschieben des Innenrohrs aus dem Führungsrohr heraus und ebenso wieder hinein fährt.

In ganz besonders raffinierter Weise ist die Schlinge derart am freien Ende des Führungsrohrs befestigt und hat die Schlinge im eingezogenen Zustand eine derartige mechanische Vorspannung, dass sie beim Herausfahren aus dem Führungsrohr sukzessive aufweitet.

Zur einfachen Handhabung des Instruments ist es von weiterem Vorteil, wenn der jeweilige Zustand der Schlinge, beispielsweise der ausgefahrene Zustand, durch Fixierung des Innenrohrs gegenüber dem Führungsrohr mittels Klemmmechanismus, vorzugsweise mittels Fixierschraube, festlegbar ist. Dadurch ist eine sichere Handhabung des Instruments gewährleistet.

Die Handhabung des Instruments ist des Weiteren dadurch begünstigt, dass ein vorderer Bereich der Sonde zumindest geringfügig gegenüber der Längsachse der Sonde abgewinkelt ist, vorzugsweise um etwa 15°. Unter einem solchen Neigungswinkel kann beispielsweise die RPE-Oberfläche unter Zugrundelegung der elastischen Eigenschaften und Kurvatur des Schlingenmaterials in einer "flachen Schicht" bearbeitet werden. Außerdem gewährleistet das erfindungsgemäße Instrument eine nicht unerhebliche Dämpfung unkontrollierter oder zu "harter" Bewegungen des Chirurgen. Eine glatte Oberfläche der Schlinge ermöglicht zudem einfaches und gleichmäßiges Gleiten auf der jeweiligen Oberfläche/Membran mit geringer Wahrscheinlichkeit von Gewebslazeration.

Eine weitere mechanische Ausgestaltung der Sonde bzw. des Führungsrohrs ist ein ovaler oder abgeflachter Querschnitt im Bereich des freien Endes. Im Rahmen einer solchen Ausgestaltung kann die dünne Schlinge in dem ovalen bzw. abgeflachten Rohr einfach gefaltet und retrahiert liegen. Nach dem Einbringen der Sonde in das Auge lässt sich die Schlinge in idealer Weise herausfahren, bis der zur Behandlung erforderliche Durchmesser erreicht ist.

Eine weitere raffinierte Maßnahme ist die Vorkehrung mindestens einer Öffnung im Führungsrohr, nahe dem freien Ende. Eine solche Öffnung dient zum Druckausgleich, nämlich zur Vermeidung von Unterdruck-/Überdruck-Situationen im Sondenbereich innerhalb des Auges. Ein freier Druckausgleich ist möglich. Der Durchgang kann quer zur Längsrichtung des Führungsrohrs verlaufen.

Des Weiteren sei angemerkt, dass das erfindungsgemäße Instrument insgesamt aus Edelstahl gefertigt sein kann. Ebenso ist es denkbar, die Bestandteile des Instruments insgesamt oder zumindest teilweise aus Kunststoff zu fertigen, beispielsweise im Rahmen eines zum einmaligen Gebrauch gedachten Instruments.

Wie bereits zuvor ausgeführt, handelt es sich bei dem erfindungsgemäßen Instrument um ein rein mechanisch wirkendes Instrument mit einfachster Konstruktion und Handhabung. Ein solches Instrument kann auch zum epiretinalen Membran-Peeling im Rahmen der Makulachirurgie verwendet werden. Dazu könnte eine Oberflächenmodifikation der Schlinge vorgenommen werden, beispielsweise durch Beschichtung des eigentlichen Schlingenmaterials mit Nanofasern oder Mikro-/Nanopartikeln, um die Wirkung der Schlinge in Bezug auf den Abrasiveffekt zu intensivieren.

Auch ist es denkbar, das erfindungsgemäße Instrument zur grundsätzlichen Mobilisation von Oberflächen bzw. Membranen zu verwenden, um dort nämlich die Mobilisierung begünstigende Reize zu setzen. Grundsätzlich sind jedwede Anwendungen des erfindungsgemäßen Instruments im Auge denkbar, wonach zumindest eine Stimulation der Zellen stattfindet. So ist auch die Behandlung von Narben, insbesondere auch das Polieren an bzw. auf der Innenfläche der Linsenkapsel möglich. Nicht zuletzt auch aufgrund des in Bezug auf die Kraftübertragung dämpfenden Charakters des Instruments ist eine universelle und dabei schonende Anwendung im Auge möglich. Der durch das Aufweiten der Schlinge auftretende flächige Effekt minimiert die Gefahr punktueller Krafteinwirkungen, so dass das erfindungsgemäße Instrument im Gegensatz zu dem Einsatz von Spatel, Gummi, etc. schonend wirkt und gleichermaßen eine große Fläche bzw. ein großes Volumen erreichbar ist.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht ein Ausführungsbeispiel eines erfindungsgemäßen Instruments mit abgewinkelter Sonde,
- Fig. 2: in einer schematischen Ansicht ein weiteres Ausführungsbeispiel eines erfindungsgemäßen Instruments mit Klemmmechanismus zur Fixierung einer Arbeitsposition vorgesehen ist und
- Fig. 3 bis 5: in vergrößerter Ansicht das distale Ende der Sonde mit ausgefahrener Schlinge in Draufsicht (Fig. 3), mit ausgefahrener Schlinge in Seitenansicht (Fig. 4) und mit eingefahrener Schlinge in Draufsicht (Fig. 5), wobei in der Sonde abgewinkelt ist und einen Durchgang zum Druckausgleich hat.

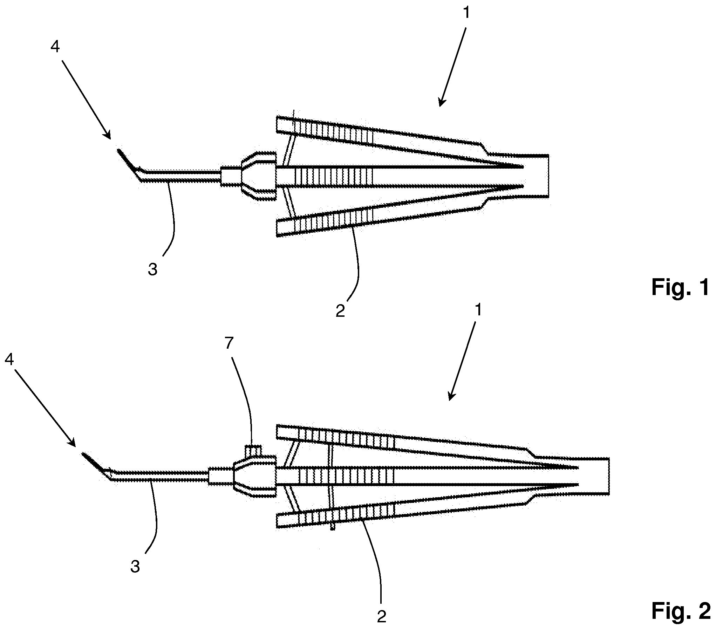

Die Fig. 1 und 2 zeigen in schematischer Ansicht zwei Ausführungsbeispiele eines erfindungsgemäßen Instruments zur Stimulation bzw. Irritation und/oder zur Abrasivbehandlung und/oder zum Polieren der Membran oder Oberfläche oder Innenfläche im menschlichen oder tierischen Auge. Das Instrument umfasst einen Handgriff 1 und ein dem Handgriff 1 zugeordnetes Betätigungsorgan 2, wobei dieses integraler Bestandteil des Handgriffs 1 ist.

An den Handgriff 1 schließt sich eine Sonde 3 an, die am freien, distalen Ende offen ist. Die Sonde 3 dient zum Einführen in das Auge.

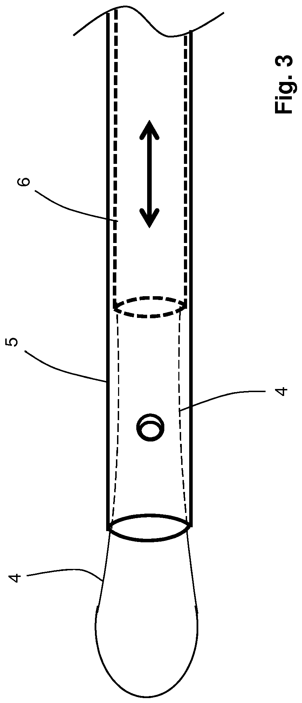

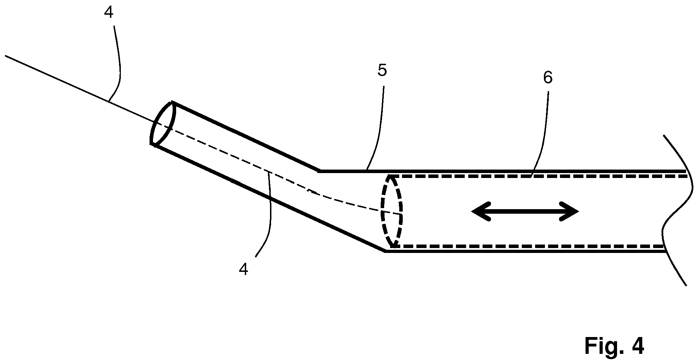

Erfindungsgemäß umfasst die Sonde 3, im Innern, eine Schlinge 4, die über das Betätigungsorgan 2 zumindest geringfügig aus der Sonde 3 heraus schiebbar und in die Sonde 3 zurück schiebbar bzw. ziehbar ist. Die Schlinge ist in den jeweiligen Zuständen in den Fig. 3, 4 und 5 gezeigt.

Bei der Schlinge 4 kann es sich um einen Metall- oder Kunststoffdraht handeln, hier im Konkreten um einen Draht aus einem kristallinen Stereoisomer von Polypropylen.

Die Schlinge 4 hat einen Durchmesser im Bereich von 0,06 bis 0,07 mm. Im ausgefahrenen Zustand ist die Schlinge 4 in etwa 3 mm lang und hat einen Innendurchmesser von etwa 1,6 mm. Ein hinreichend großer Arbeitsbereich ist damit garantiert.

Die Sonde 3 umfasst ein äußeres Führungsrohr 5 und ein endseitig die Schlinge 4 tragendes Innenrohr 6, wobei das Innenrohr 6 durch Betätigung des Betätigungsorgans 2 innerhalb des Führungsrohrs 5 verschiebbar ist. Durch diese Verschiebung ist die Schlinge 4 aus dem Führungsrohr 5 heraus und in das Führungsrohr 5 hinein verlagerbar.

Gemäß der Darstellung in Fig. 2 ist ein Klemmmechanismus 7 in Form einer Fixierschraube vorgesehen, der eine Fixierung der jeweiligen Position der Schlinge 4 ermöglicht.

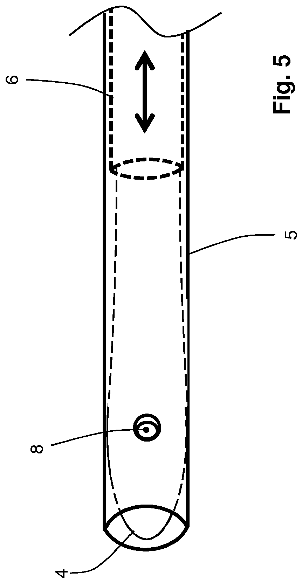

Schließlich zeigen die Fig. 3, und 5 die Vorkehrung eines quer zur Längsachse des Führungsrohrs 5 verlaufenden Durchgangs 8, der zum Druckausgleich, nämlich zur Vermeidung von Unter-/Überdruck im Arbeitsbereich der Sonde 3, dient.

Hinsichtlich weiterer Merkmale, die sich den Figuren nicht entnehmen lassen, sei zur Vermeidung von Wiederholungen auf den allgemeinen Teil der Beschreibung sowie auf die beigefügten Patentansprüche verwiesen.

Schließlich sei ausdrücklich darauf hingewiesen, dass die voranstehend beschriebenen Ausführungsbeispiele des erfindungsgemäßen Instruments lediglich zur Erörterung der beanspruchten Lehre dienen, diese jedoch nicht auf die Ausführungsbeispiele einschränken.

### Bezugszeichenliste

- 1: Handgriff
- 2: Betätigungsorgan
- 3: Sonde
- 4: Schlinge
- 5: äußeres Führungsrohr
- 6: Innenrohr
- 7: Klemmmechanismus, Fixierschraube
- 8: Durchgang

## Patentansprüche

1. Instrument zur Stimulation bzw. Irritation und/oder zur Abrasivbehandlung und/oder zum Polieren einer Membran oder Oberfläche oder Innenfläche im menschlichen oder tierischen Auge, mit einem ein Betätigungsorgan (2) umfassenden Handgriff (1) und einer sich an den Handgriff (1) anschließenden, am freien, distalen Ende offenen Sonde (3) zum Einführen in das Auge, wobei die Sonde (3), im Inneren, eine Schlinge (4) umfasst, die über das Betätigungsorgan (2) zumindest geringfügig aus der Sonde (3) heraus schiebbar und in die Sonde (3) zurück schiebbar bzw. ziehbar ist,
**dadurch gekennzeichnet, dass** die Sonde (3) ein äußeres Führungsrohr (5) und ein endseitig die Schlinge tragendes Innenrohr (6) umfasst und dass das Innenrohr (6) durch Betätigung des Betätigungsorgans (2) innerhalb des Führungsrohres (5) verschiebbar ist, wodurch die Schlinge (4) aus dem Führungsrohr (5) heraus und in das Führungsrohr (5) hinein verlagerbar ist.

2. Instrument nach Anspruch 1, **dadurch gekennzeichnet, dass** die Schlinge (4) als dünner Metall- oder Kunststoffdraht ausgeführt ist.

3. Instrument nach Anspruch 1 oder 2, **dadurch gekennzeichnet, dass** die Schlinge (4) aus einem kristallinen Stereoisomer von Polypropylen besteht.

4. Instrument nach einem der Ansprüche 1 bis 3, **dadurch gekennzeichnet, dass** die Schlinge (4) einen Materialdurchmesser von etwa 0,06 bis 0,07 mm hat.

5. Instrument nach einem der Ansprüche 1 bis 4, **dadurch gekennzeichnet, dass** die Schlinge (4) im herausgeschobenen Zustand vom freien Ende der Sonde (3) aus eine Länge von 2 bis 4 mm, vorzugsweise 3 mm, und einen Innendurchmesser von 1 bis 2 mm, vorzugsweise 1,6 mm, hat.

6. Instrument nach einem der Ansprüche 1 bis 5, **dadurch gekennzeichnet, dass** die Schlinge (4) derart am freien Ende des Führungsrohres (5) befestigt ist und im eingezogenen Zustand eine derarte mechanische Vorspannung hat, dass sie sich beim Herausschieben aus dem Führungsrohr (5) sukzessive aufweitet.

7. Instrument nach einem der Ansprüche 1 bis 6, **dadurch gekennzeichnet, dass** der jeweilige Zustand der Schlinge (4) durch Fixierung des Innenrohrs (6) gegenüber dem Führungsrohr (5) mittels Klemmmechanismus (7), vorzugsweise mittels Fixierschraube, festlegbar ist.

8. Instrument nach einem der Ansprüche 1 bis 7, **dadurch gekennzeichnet, dass** ein vorderer Bereich der Sonde (3) zumindest geringfügig gegenüber der Längsachse der Sonde (3) abgewinkelt ist, vorzugsweise um etwa 15°.

9. Instrument nach einem der Ansprüche 1 bis 8, **dadurch gekennzeichnet, dass** die Sonde (3) bzw. das Führungsrohr (5) im Bereich ihres/seines freien Endes einen ovalen oder abgeflachten Querschnitt aufweist.

10. Instrument nach einem der Ansprüche 1 bis 9, **dadurch gekennzeichnet, dass** im Führungsrohr (5), nahe dem freien Ende, mindestens ein zum Druckausgleich dienender, im Wesentlichen quer zur Längsachse des Führungsrohrs (5) verlaufender Durchgang (7) ausgebildet ist.

11. Instrument nach einem der Ansprüche 1 bis 10, **dadurch gekennzeichnet, dass** die einzelnen Bestandteile aus Edelstahl und/oder Kunststoff gefertigt sind.

## Claims

1. Instrument for stimulating and/or irritating and/or abrasively treating and/or polishing a membrane or surface or inner face in the human or animal eye, having a handle (1) which surrounds an actuation member (2) and a probe (3) which adjoins the handle (1) and which is open at the free, distal end for introduction into the eye, wherein the probe (3) comprises at the inner side a loop (4) which via the actuation member (2) can be pushed at least slightly out of the probe (3) and can be pushed or pulled back into the probe (3),
**characterised in that** the probe (3) has an outer guiding pipe (5) and an inner pipe (6) which carries the loop at the end side and **in that** the inner pipe (6) can be displaced by actuating the actuation member (2) inside the guiding pipe (5), whereby the loop (4) can be displaced out of the guiding pipe (5) and into the guiding pipe (5).

2. Instrument according to claim 1, **characterised in that** the loop (4) is constructed as a thin metal or plastics material wire.

3. Instrument according to claim 1 or claim 2, **characterised in that** the loop (4) comprises a crystalline stereoisomer of polypropylene.

4. Instrument according to any one of claims 1 to 3, **characterised in that** the loop (4) has a material diameter of approximately 0.06 to 0.07 mm.

5. Instrument according to any one of claims 1 to 4, **characterised in that** the loop (4) in the pushed-out state from the free end of the probe (3) has a length of from 2 to 4 mm, preferably 3 mm, and an inner diameter of from 1 to 2 mm, preferably 1.6 mm.

6. Instrument according to any one of claims 1 to 5, **characterised in that** the loop (4) is secured to the free end of the guiding pipe (5) in such a manner and in the retracted state has such a mechanical pretensioning that it successively expands when pushed out of the guiding pipe (5).

7. Instrument according to any one of claims 1 to 6, **characterised in that** the respective state of the loop (4) can be determined by fixing the inner pipe (6) with respect to the guiding pipe (5) by means of a clamping mechanism (7), preferably by means of a fixing screw.

8. Instrument according to any one of claims 1 to 7, **characterised in that** a front region of the probe (3) is at least slightly angled with respect to the longitudinal axis of the probe (3), preferably by approximately 15°.

9. Instrument according to any one of claims 1 to 8, **characterised in that** the probe (3) or the guiding pipe (5) has in the region of the free end thereof an oval or flattened cross-section.

10. Instrument according to any one of claims 1 to 9, **characterised in that** in the guiding pipe (5), close to the free end, at least one passage (7) which is used for pressure compensation and which extends substantially transversely relative to the longitudinal axis of the guiding pipe (5) is formed.

11. Instrument according to any one of claims 1 to 10, **characterised in that** the individual components are produced from high-grade steel and/or plastics material.

## Revendications

1. Instrument pour la stimulation ou l'irritation et/ou pour le traitement abrasif et/ou pour le polissage d'une membrane ou une surface ou une surface interne dans l'œil humain ou animal, avec une poignée (1) comprenant un organe d'actionnement (2) et une sonde (3), se raccordant à la poignée (1), ouverte à l'extrémité distale libre, destinée à être introduite dans l'œil, la sonde (3) comprenant, à l'intérieur, une boucle (4) qui peut être poussée, par l'intermédiaire de l'organe d'actionnement (2), au moins faiblement hors de la sonde (3) et peut être poussée ou rétractée à nouveau dans la sonde (3),
**caractérisé en ce que** la sonde (3) comprend un tube de guidage externe (5) et un tube interne (6) supportant la boucle à son extrémité et **en ce que** le tube interne (6) peut être coulissé, par l'actionnement de l'organe d'actionnement (2), à l'intérieur du tube de guidage (5), ce qui permet de déplacer la boucle (4) hors du tube de guidage (5) et vers l'intérieur du tube de guidage (5).

2. Instrument selon la revendication 1, **caractérisé en ce que** la boucle (4) est réalisée comme un fil mince métallique ou en matière plastique.

3. Instrument selon la revendication 1 ou 2, **caractérisé en ce que** la boucle (4) est constituée d'un stéréo-isomère cristallin de polypropylène.

4. Instrument selon l'une des revendications 1 à 3, **caractérisé en ce que** la boucle (4) présente un diamètre de matériau d'environ 0,06 à 0,07 mm.

5. Instrument selon l'une des revendications 1 à 4, **caractérisé en ce que** la boucle (4) présente, dans l'état sorti, à partir de l'extrémité libre de la sonde (3), une longueur de 2 à 4 mm, de préférence de 3 mm, et un diamètre intérieur de 1 à 2 mm, de préférence de 1,6 mm.

6. Instrument selon l'une des revendications 1 à 5, **caractérisé en ce que** la boucle (4) est fixée à l'extrémité libre du tube de guidage (5) et présente, dans l'état rétracté, une précontrainte mécanique de façon à ce qu'elle s'élargisse de manière successive lors de la poussée hors du tube de guidage (5).

7. Instrument selon l'une des revendications 1 à 6, **caractérisé en ce que** l'état respectif de la boucle (4) peut être fixé par la fixation du tube interne (6) par rapport au tube de guidage (5) au moyen d'un mécanisme de serrage (7), de préférence au moyen d'une vis de fixation.

8. Instrument selon l'une des revendications 1 à 7, **caractérisé en ce qu'**une partie avant de la sonde (3) est inclinée au moins faiblement par rapport à l'axe longitudinal de la sonde (3), de préférence d'environ 15°.

9. Instrument selon l'une des revendications 1 à 8, **caractérisé en ce que** la sonde (3) ou le tube de guidage (5) présente, au niveau de son extrémité libre, une section transversale ovale ou aplatie.

10. Instrument selon l'une des revendications 1 à 9, **caractérisé en ce que**, dans le tube de guidage (5), près de l'extrémité libre, est réalisé au moins un passage (7) permettant l'équilibrage de la pression, s'étendant globalement transversalement par rapport à l'axe longitudinal du tube de guidage (5).

11. Instrument selon l'une des revendications 1 à 10, **caractérisé en ce que** les différents composants sont constitués d'acier inoxydable et/ou de matière plastique.
